(19) Europäisches Patentamt
European Patent Office
Office européen des brevets

(11) EP 3 229 491 B1

(12) **EUROPEAN PATENT SPECIFICATION**

(45) Date of publication and mention of the grant of the patent:
**09.04.2025 Bulletin 2025/15**

(21) Application number: **15865756.9**

(22) Date of filing: **30.11.2015**

(51) International Patent Classification (IPC):
***C08L 83/04*** (2006.01) ***A61B 8/12*** (2006.01)
***A61B 8/13*** (2006.01) ***A61B 8/14*** (2006.01)
***H04R 19/00*** (2006.01)

(52) Cooperative Patent Classification (CPC):
(C-Sets available)
**C08K 5/56; A61B 8/12; A61B 8/13; A61B 8/14; C08K 3/22; C08L 83/00; C08L 83/04; H04R 19/00;**
C08G 77/12; C08G 77/20 (Cont.)

(86) International application number:
**PCT/JP2015/083536**

(87) International publication number:
**WO 2016/088699 (09.06.2016 Gazette 2016/23)**

(54) **COMPOSITION FOR ACOUSTIC WAVE PROBE, SILICONE RESIN FOR ACOUSTIC WAVE PROBE USING SAME, ACOUSTIC WAVE PROBE, ULTRASONIC PROBE, ACOUSTIC WAVE MEASUREMENT DEVICE, ULTRASONIC DIAGNOSTIC DEVICE, PHOTOACOUSTIC WAVE MEASUREMENT DEVICE, AND ULTRASONIC ENDOSCOPE**

ZUSAMMENSETZUNG FÜR EINE AKUSTIKWELLENSONDE, SILIKONHARZ FÜR EINE AKUSTIKWELLENSONDE DAMIT, AKUSTIKWELLENSONDE, ULTRASCHALLSONDE, AKUSTIKWELLENMESSVORRICHTUNG, ULTRASCHALLDIAGNOSEVORRICHTUNG, PHOTOAKUSTIKWELLENMESSVORRICHTUNG UND ULTRASCHALLENDOSKOP

COMPOSITION POUR LE SONDAGE D'ONDES ACOUSTIQUES, RÉSINE DE SILICIUM POUR SONDAGE D'ONDES ACOUSTIQUES QUI L'UTILISE, SONDE D'ONDES ACOUSTIQUES ET SONDE ULTRASONIQUE, ET DISPOSITIF DE MESURE D'ONDES ACOUSTIQUES, DISPOSITIF DE DIAGNOSTIC À ULTRASONS, DISPOSITIF DE MESURE D'ONDES PHOTOACOUSTIQUES ET ENDOSCOPE ULTRASONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.12.2014 JP 2014243074**
**06.11.2015 JP 2015218499**

(43) Date of publication of application:
**11.10.2017 Bulletin 2017/41**

(73) Proprietor: **FUJIFILM Corporation**
**Tokyo 106-8620 (JP)**

(72) Inventors:
- **NAGAI, Takayasu**
**Kanagawa 258-8577 (JP)**
- **NAKAI, Yoshihiro**
**Kanagawa 258-8577 (JP)**
- **OSAWA, Atsushi**
**Kanagawa 258-8577 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 930 213**
**WO-A1-2014/088115**
**WO-A1-2017/002746**
**JP-A- 2009 240 782**
**US-A- 5 786 414**
**US-A1- 2009 243 436**
**US-A1- 2010 213 415**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **PARIMAL NAIK: "PREPARATION OF HIGH FLUX HYDROPHOBIC COMPOSITE MEMBRANES", THESIS FOR: PH.D IN BIO-SCIENCE ENGINEERING, 20 August 2016 (2016-08-20), XP055420503, Retrieved from the Internet <URL:https://www.researchgate.net/profile/Parimal_Naik3/publication/306322667_PREPARATION_OF_HIGH_FLUX_HYDROPHOBIC_COMPOSITE_MEMBRANES/links/57b8072f08ae6f1737650b28/PREPARATION-OF-HIGH-FLUX-HYDROPHOBIC-COMPOSITE-MEMBRANES.pdf> [retrieved on 20171031], DOI: 10.13140/RG.2.2.29647.69283/1**
- **DATABASE WPI Week 200941, Derwent World Patents Index; AN 2009-K12767**


(52) Cooperative Patent Classification (CPC): (Cont.)

C-Sets
**C08L 83/04, C08L 83/00, C08K 5/56, C08K 3/22**

## Description

## BACKGROUND OF THE INVENTION

1. Field of the Invention

The present invention relates to the use of a composition in the production of an acoustic wave probe, the acoustic wave probe, and an ultrasound probe. Furthermore, the present invention relates to an acoustic wave measurement apparatus, an ultrasound diagnostic apparatus, a photoacoustic wave measurement apparatus, and an ultrasound endoscope.

2. Description of the Related Art

In the acoustic wave measurement apparatus, an acoustic wave probe is used which irradiates an object or an area (hereinafter, simply referred to as the object) with an acoustic wave, receives a reflected wave (echo) thereof, and outputs a signal. An electrical signal converted from the reflected wave which has been received by this acoustic wave probe is displayed as an image. Accordingly, the interior of the object is visualized and observed.

An appropriate frequency such as an ultrasonic wave or a photoacoustic wave is selected as the acoustic wave in accordance with a test object, measurement conditions, and the like.

For example, the ultrasound diagnostic apparatus transmits an ultrasonic wave to the interior of a test object, receives the ultrasonic wave reflected by the tissues inside the test object, and displays the received ultrasonic wave as an image. The photoacoustic wave measurement apparatus receives an acoustic wave radiated from the interior of a test object due to a photoacoustic effect, and displays the received acoustic wave as an image. The photoacoustic effect is a phenomenon in which an acoustic wave (typically an ultrasonic wave) is generated through thermal expansion after a test object absorbs an electromagnetic wave and generates heat when the test object is irradiated with an electromagnetic wave pulse of visible light, near infrared light, microwave, or the like.

The acoustic wave measurement apparatus performs transmission and reception of an acoustic wave on a living body which is a test object. Therefore, it is necessary to fulfill requirements such as consistency in the acoustic impedance within a living body or decrease in acoustic attenuation.

For example, a probe for an ultrasound diagnostic apparatus (also referred to as an ultrasound probe) which is a kind of acoustic wave probe includes a piezoelectric element which transmits and receives an ultrasonic wave and an acoustic lens which is a portion coming into contact with a living body. An ultrasonic wave oscillated from the piezoelectric element is incident on the living body after being transmitted through the acoustic lens. In a case where the difference between acoustic impedance (density x acoustic velocity) of the acoustic lens and acoustic impedance of the living body is large, the ultrasonic wave is reflected by the surface of the living body, and therefore, it is difficult to obtain high resolution since the ultrasonic wave is not efficiently incident on the living body. In addition, it is desirable that ultrasonic attenuation of the acoustic lens is low in order to transmit and receive the ultrasonic wave with high sensitivity.

For this reason, a silicone resin of which the acoustic impedance is close to the acoustic impedance ($1.4 \times 10^6$ to $1.7 \times 10^6$ $kg/m^2/sec$) of a living body and which has a low ultrasonic attenuation is mainly used as a material of the acoustic lens.

For example, addition of inorganic filler and thermoplastic resin powder such as nylon powder to silicone rubber as a composition for an acoustic lens is proposed in JP1987-011897A (JP-S62-011897A) and US 2009/243436 A1.

In addition, an acoustic lens requires mechanical strength to withstand long-term use in order to use the acoustic lens in a state of abutting on a test object. For this reason, in JP2005-125071A, a composition containing silicone rubber, powder such as ytterbium oxide, and silica particles is proposed as a composition for an acoustic lens which satisfies the characteristics of the acoustic lens (such as acoustic impedance, ultrasonic attenuation, and mechanical strength).

## SUMMARY OF THE INVENTION

A silicone resin alone is soft and has a low mechanical strength. For this reason, formulation of an inorganic filler or a vinyl group-containing resin (also referred to as a reinforcing agent) is performed while setting the molecular weight of a vinyl silicone resin at both terminals to be large, for the purpose of improving the hardness and the mechanical strength. However, in a case where the required mechanical strength is to be achieved, there is a problem in that the amount of the inorganic filler or the vinyl group-containing resin added to the silicone resin necessarily becomes large and the acoustic attenuation of a silicone resin contrarily increases.

For this reason, it has been difficult for conventional silicone resins to satisfy all conditions such as high resin hardness and mechanical strength and reduced acoustic attenuation to a high level.

Accordingly, the present invention has been made in consideration of the above-described circumstances, and

an object of the present invention is to provide the use of a composition in the production of an acoustic wave probe which can significantly improve the hardness and the mechanical strength (tensile strength at break, tensile elongation at break, tear strength, and abrasion resistance) of a silicone resin while maintaining a low acoustic attenuation, an acoustic wave probe, an acoustic wave measurement apparatus, and an ultrasound diagnostic apparatus.

In addition, another object of the present invention is to provide an ultrasound probe in which it is possible to use capacitive micromachined ultrasonic transducers (cMUT) with insufficient sensitivity as an ultrasonic diagnostic transducer array. In addition, the used composition for an acoustic wave probe provides an acoustic wave probe in which it is possible to improve the sensitivity of a photoacoustic wave measurement apparatus in which observation of deeper regions of a human body is difficult due to low sensitivity since the amount of ultrasonic wave generated by a photoacoustic wave is small. In addition, the used composition for an acoustic wave probe improves the sensitivity of an ultrasound endoscope in which the sensitivity is low since a signal line cable is longer than a cable for a body surface and it is difficult to improve the sensitivity due to a structure, physical characteristics, and a process aptitude.

The present inventors have studied inorganic compounds to be added to a silicone resin composition for a composition for an acoustic wave probe. As a result, they have found that it is possible to solve the above-described problems by containing a specific inorganic compound particle as claimed which has a particle diameter within a specific range, and the present invention has been completed based on this finding.

Unless otherwise specified in the description of the present specification, in a case where there are a plurality of groups having the same reference numerals as each other in General Formulas representing compounds, these may be the same as or different from each other and a group (for example, an alkyl group) specified by each group may further have a substituent. In addition, the "Si-H group" means a group having three bonds on a silicon atom. However, the description of these bonds is omitted and simplified.

In addition, "to" in the present specification is used for the meaning of including numerical values before and after "to" described as lower limit values and upper limit values.

Unless otherwise specified, the mass average molecular weight in the present specification refers to a value (in terms of polystyrene) measured through gel permeation chromatography (GPC).

According to the present invention, it is possible to provide the use of a composition in the production of an acoustic wave probe which can significantly improve the hardness and the mechanical strength (tensile strength at break, tensile elongation at break, tear strength, and abrasion resistance) of a silicone resin while maintaining a low acoustic (particularly preferably ultrasonic) attenuation, the acoustic wave probe using the composition for an acoustic wave probe, an acoustic wave measurement apparatus, and an ultrasound diagnostic apparatus.

In addition, it is possible to provide an ultrasound probe in which cMUT is used as an ultrasonic diagnostic transducer array, and to use a silicone resin for an acoustic wave probe which can improve the sensitivity of the photoacoustic wave measurement apparatus and the ultrasound endoscope.

It is considered that such an effect is obtained since inorganic compound particles having a small average primary particle diameter function as stoppers in a case where mechanical stress is applied to the silicone resin in an acoustic wave probe. The distance between the particles becomes shorter in a case where the average primary particle diameter is small. Therefore, the inorganic compound particles more favorably exhibit functions of stoppers and significantly improve the tear strength of the silicone resin.

As a result, it is considered that the increase in acoustic attenuation is suppressed and the hardness and the mechanical strength (tensile strength at break, tensile elongation at break, tear strength, and abrasion resistance) of the silicone resin for an acoustic wave probe are improved.

The above-described characteristics and advantages and other characteristics and advantages of the present invention become clearer in the following descriptions with reference to the accompanying drawing.

## BRIEF DESCRIPTION OF THE DRAWING

Fig. 1 is a perspective transparent view of an example of a convex ultrasound probe which is an embodiment of an acoustic wave probe.

## DESCRIPTION OF THE PREFERRED EMBODIMENTS

<<Composition for Acoustic Wave Probe>>

A composition for an acoustic wave probe of the present invention (hereinafter, also simply referred to as a composition) is a composition for an acoustic wave probe containing a polysiloxane mixture containing at least polysiloxane having a vinyl group, polysiloxane having two or more Si-H groups in a molecular chain, and one or more inorganic compound particles, in which the average primary particle diameter of the inorganic compound particles is less than 25 nm and the inorganic compound particles are selected from the group consisting of magnesium oxide, titanium oxide, iron

oxide, zinc oxide, zirconium oxide, barium oxide, tin oxide, and ytterbium oxide, and wherein the inorganic compound particles are subjected to surface treatment using a silane compound.

The content of inorganic compound particles in 100 parts by mass in total of the polysiloxane mixture is preferably 10 to 60 parts by mass, more preferably 15 to 50 parts by mass, and still more preferably 20 to 40 parts by mass.

In addition, the content of polysiloxane having a vinyl group in 100 parts by mass in total of the polysiloxane mixture is preferably 10 to 99.4 parts by mass and the content of polysiloxane having two or more Si-H groups in a molecular chain is preferably 0.5 to 90 parts by mass. The content of polysiloxane having a vinyl group is more preferably 50 to 90 parts by mass and the content of polysiloxane having two or more Si-H groups in a molecular chain is more preferably 1 to 50 parts by mass.

The polysiloxane mixture refers to a mixture which does not contain a catalyst for crosslinking and polymerizing (vulcanizing) the polysiloxane having a vinyl group and the polysiloxane having two or more Si-H groups in a molecular chain. Accordingly, the polysiloxane mixture contains inorganic compound particles but no catalyst.

In addition, 100 parts by mass in total of the polysiloxane mixture means that the total of the components individually contained in the polysiloxane mixture is 100 parts by mass.

Each polysiloxane described above which is contained in the polysiloxane mixture may be any polysiloxane as long as it has a vinyl group or two or more Si-H groups in a molecular chain. However, in the present invention, polyorganosiloxane (A) having a vinyl group and polyorganosiloxane (B) having two or more Si-H groups in a molecular chain are preferable.

Accordingly, in the present invention, a composition is preferable which contains at least the polyorganosiloxane (A) having a vinyl group, the polyorganosiloxane (B) having two or more Si-H groups in a molecular chain, and inorganic compound particles (C) in the polyorganosiloxane mixture.

In the detailed description below, a polysiloxane mixture will be described which is a preferred embodiment and contains the polyorganosiloxane (A) having a vinyl group and the polyorganosiloxane (B) having two or more Si-H groups in a molecular chain. However, each polysiloxane contained in the polysiloxane mixture is not limited to the polysiloxane (A) and the polysiloxane (B).

<Polyorganosiloxane (A) having Vinyl Group>

The polyorganosiloxane (A) having a vinyl group (hereinafter, also simply referred to as a polyorganosiloxane (A)) used in the present invention has two or more vinyl groups in a molecular chain.

Examples of the polyorganosiloxane (A) having a vinyl group include 8 polyorganosiloxane (a) having a vinyl group at least at both terminals of a molecular chain (hereinafter, also simply referred to as polyorganosiloxane (a)) or polyorganosiloxane (b) having at least two $-O-Si(CH_3)_2(CH=CH_2)$ in a molecular chain (hereinafter, also simply referred to as a polyorganosiloxane (b)). Among these, the polyorganosiloxane (a) having a vinyl group at least at both terminals of a molecular chain is preferable.

The polyorganosiloxane (a) is preferably linear and the polyorganosiloxane (b) is preferably polyorganosiloxane (b) in which $-O-Si(CH_3)_2(CH=CH_2)$ is bonded to a Si atom constituting a main chain.

The polyorganosiloxane (A) having a vinyl group is subjected to hydrosilylation through a reaction with the polyorganosiloxane (B) having two or more Si-H groups in the presence of, for example, a platinum catalyst. A cross-linked (vulcanized) structure is formed through this hydrosilylation reaction (addition reaction).

The content of the vinyl group of the polyorganosiloxane (A) is not particularly limited. The content of the vinyl group is, for example, preferably 0.01 to 5 mol% and more preferably 0.05 to 2 mol% from the viewpoint of forming a sufficient network between components contained in a composition for an acoustic wave probe.

Here, the content of the vinyl group is mol% of a vinyl group-containing siloxane unit in a case where all units constituting the polyorganosiloxane (A) is set to 100 mol%. One vinyl group-containing siloxane unit has one to three vinyl groups. Among these, one vinyl group is preferable with respect to one vinyl group-containing siloxane unit. For example, in a case where all Si atoms of Si in a Si-O unit and terminal which constitute a main chain has at least one vinyl group, the content becomes 100 mol%.

In addition, the polyorganosiloxane (A) preferably has a phenyl group and the content of the phenyl group of the polyorganosiloxane (A) is not particularly limited. The content of the phenyl group of the polyorganosiloxane (A) is, for example, preferably 1 to 80 mol% and more preferably 2 to 40 mol% from the viewpoint of mechanical strength in a case of using the silicone resin for an acoustic wave probe.

Here, the content of the phenyl group is mol% of a phenyl group-containing siloxane unit in a case where all units constituting the polyorganosiloxane (A) is set to 100 mol%. One phenyl group-containing siloxane unit has one to three phenyl groups. Among these, two phenyl groups are preferable with respect to one phenyl group-containing siloxane unit. For example, in a case where all Si atoms of Si in a Si-O unit and terminal which constitute a main chain has at least one phenyl group, the content becomes 100 mol%.

The unit refers to Si in a Si-O unit and terminal which constitutes a main chain.

The degree of polymerization and the specific gravity are not particularly limited. The degree of polymerization is preferably 200 to 3,000 and more preferably 400 to 2,000, and the specific gravity is preferably 0.9 to 1.1 from the viewpoint of improving the mechanical strength, the hardness, the chemical stability, or the like of an obtained silicone resin for an acoustic wave probe (hereinafter, also simply referred to as a silicone resin).

The mass average molecular weight of the polyorganosiloxane having a vinyl group is preferably 20,000 to 200,000, more preferably 40,000 to 150,000, and still more preferably 45,000 to 120,000 from the viewpoint of the mechanical strength, the hardness, and easiness of processing.

The mass average molecular weight can be measured using, for example, TOLUENE (manufactured by Shonan Wako Junyaku K.K.) as an eluent, TSKgel (registered trademark), G3000HXL + TSKgel (registered trademark), and G2000HXL as columns, and a RI detector under the conditions of a temperature of 23°C and a flow rate of 1 mL/min after preparing a GPC apparatus HLC-8220 (manufactured by TOSOH CORPORATION).

The kinematic viscosity at 25°C is preferably 1 x $10^{-5}$ to 10 $m^2/s$, more preferably 1 x $10^{-4}$ to 1 $m^2/s$, and still more preferably 1 x $10^{-3}$ to 0.5 $m^2/s$.

The kinematic viscosity can be measured and obtained at a temperature of 25°C using Ubbelohde-type viscometer (for example, a trade name of SU manufactured by SIBATA SCIENTIFIC TECHNOLOGY LTD.) in compliance with JIS Z8803.

Polyorganosiloxane represented by the following General Formula (A) is preferable as the polyorganosiloxane (a) having a vinyl group at least at both terminals of a molecular chain.

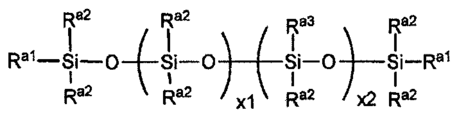
(A)

In General Formula (A), $R^{a1}$ represents a vinyl group and $R^{a2}$ and $R^{a3}$ each independently represent an alkyl group, a cycloalkyl group, an alkenyl group, or an aryl group. x1 and x2 each independently represent an integer of 1 or more. Here, a plurality of $R^{a2}$'s and a plurality of $R^{a3}$'s may be the same as or different from each other. In addition, each of the groups of $R^2$ and $R^{a3}$ may further have a substituent.

The number of carbon atoms in an alkyl group in $R^{a2}$ and $R^{a3}$ is preferably 1 to 10, more preferably 1 to 4, still more preferably 1 or 2, and particularly preferably 1. Examples of the alkyl group include methyl, ethyl, n-propyl, isopropyl, n-butyl, isobutyl, n-hexyl, n-octyl, 2-ethylhexyl, and n-decyl.

The number of carbon atoms in a cycloalkyl group in $R^{a2}$ and $R^{a3}$ is preferably 3 to 10, more preferably 5 to 10, and still more preferably 5 or 6. In addition, the cycloalkyl group is preferably a 3-membered ring, a 5-membered ring, or a 6-membered ring, and more preferably a 5-membered ring or a 6-membered ring. Examples of the cycloalkyl group include cyclopropyl, cyclopentyl, and cyclohexyl.

The number of carbon atoms in an alkenyl group in $R^{a2}$ and $R^{a3}$ is preferably 2 to 10, more preferably 2 to 4, and still more preferably 2. Examples of the alkenyl group include vinyl, allyl, and butenyl.

The number of carbon atoms in an aryl group in $R^{a2}$ and $R^{a3}$ is preferably 6 to 12, more preferably 6 to 10, and still more preferably 6 to 8. Examples of the aryl group include phenyl, tolyl, and naphthyl.

The alkyl group, the cycloalkyl group, the alkenyl group, and the aryl group may have a substituent. Examples of such a substituent include a halogen atom, an alkyl group, a cycloalkyl group, an alkenyl group, an aryl group, an alkoxy group, an aryloxy group, an alkylthio group, an arylthio group, a silyl group, and a cyano group.

Examples of the group having a substituent include a halogenated alkyl group.

$R^{a2}$ and $R^{a3}$ are preferably an alkyl group, an alkenyl group, or an aryl group, more preferably an alkyl group having 1 to 4 carbon atoms, a vinyl group, or a phenyl group, and still more preferably a methyl group, a vinyl group, or a phenyl group.

Among these, $R^{a2}$ is preferably a methyl group, and $R^{a3}$ is preferably a methyl group, a vinyl group, or a phenyl group, more preferably a methyl group, or a phenyl group, and particularly preferably a methyl group. In addition, it is preferable that both $R^{a2}$s in the repetition of x1 are phenyl groups.

x1 is preferably an integer between 200 to 3,000 and more preferably an integer between 400 to 2,000.

x2 is preferably an integer between 1 to 3,000, more preferably an integer between 1 to 1,000, still more preferably an integer between 40 to 1,000, and particularly preferably an integer between 40 to 700.

In addition, as another embodiment, x1 is preferably an integer between 1 to 3,000 and more preferably an integer between 5 to 1,000.

Examples of the polyorganosiloxane having a vinyl group at least at both terminals of a molecular chain include DMS series (for example, DMS-V31, DMS-V31S15, DMS-V33, DMS-V35, DMS-V35R, DMS-V41, DMS-V42, DMS-V46, DMS-V51, and DMS-V52), PDV series (for example, PDV-0341, PDV-0346, PDV-0535, PDV-0541, PDV-1631, PDV-1635, PDV-1641, and PDV-2335), PMV-9925, PVV-3522, FMV-4031, AND EDV-2022 which are all trade names

manufactured by GELEST, INC..

Fumed silica is formulated into DMS-V31S15, and therefore, kneading using a special device is unnecessary.

The polyorganosiloxane (A) having a vinyl group in the present invention may be used singly, or two or more thereof may be used in combination.

<Polyorganosiloxane (B) having Two or More Si-H Groups in Molecular Chain>

The polyorganosiloxane (B) having two or more Si-H groups in a molecular chain used in the present invention (hereinafter, also simply referred to as polyorganosiloxane (B)) has two or more Si-H groups in a molecular chain.

In a case where there are two or more Si-H groups in a molecular chain, it is possible to crosslink polyorganosiloxane having at least two polymerizable unsaturated groups.

There is a linear structure and a branched structure in polyorganosiloxane (B), and the linear structure is preferable.

The mass average molecular weight of a linear structure is preferably 500 to 100,000 and more preferably 1,500 to 50,000 from the viewpoint of the mechanical strength and the hardness.

The polyorganosiloxane (B) which has a linear structure and two or more Si-H groups in a molecular chain is preferably polyorganosiloxane represented by the following General Formula (B).

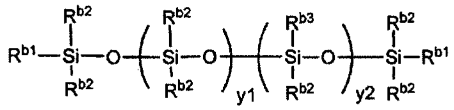

(B)

In General Formula (B), $R^{b1}$ to $R^{b3}$ each independently represent a hydrogen atom, an alkyl group, a cycloalkyl group, an alkenyl group, an aryl group, or $-O-Si(Rb^5)_2(R^{b4})$. $R^{b4}$ and $R^{b5}$ each independently represent a hydrogen atom, an alkyl group, a cycloalkyl group, an alkenyl group, or an aryl group. y1 and y2 each independently represent an integer of 1 or more. Here, a plurality of $R^{b1}$'s, a plurality of $R^{b2}$'s a plurality of $R^{b3}$'s, a plurality of $R^{b4}$'s, and a plurality of $R^{b5}$'s may be the same as or different from each other. In addition, each of the groups of $R^{b1}$ to $R^{b5}$ may further be substituted with a substituent. However, there are two or more Si-H groups in a molecular chain.

An alkyl group, a cycloalkyl group, an alkenyl group, and an aryl group in $R^{b1}$ to $R^{b3}$ are synonymous with an alkyl group, a cycloalkyl group, an alkenyl group, and an aryl group in $R^{a2}$ and $R^{a3}$, and preferred ranges thereof are also the same as each other.

An alkyl group, a cycloalkyl group, an alkenyl group, and an aryl group in $R^{b4}$ and $R^{b5}$ of $-O-Si(R^{b5})_2(R^{b4})$ are synonymous with an alkyl group, a cycloalkyl group, an alkenyl group, and an aryl group in $R^{b1}$ to $R^{b3}$, and preferred ranges thereof are also the same as each other.

$R^{b1}$ to $R^{b3}$ are preferably a hydrogen atom, an alkyl group, an alkenyl group, an aryl group, or $-O-Si(R^{b5})_2(R^{b4})$ and more preferably a hydrogen atom, an alkyl group having 1 to 4 carbon atoms, a vinyl group, a phenyl group, or $-O-Si(CH_3)_2H$.

Among these, $R^{b1}$ and $R^{b2}$ are preferably a hydrogen atom, an alkyl group, an alkenyl group, or an aryl group, more preferably a hydrogen atom or an alkyl group, and still more preferably a hydrogen atom or a methyl group.

$R^{b3}$ is preferably a hydrogen atom, an alkyl group, an alkenyl group, an aryl group, or $-O-Si(R^{b5})_2(R^{b4})$ and more preferably a hydrogen atom or $-O-Si(CH_3)_2H$.

y1 and y2 are preferably an integer between 1 to 2,000, more preferably an integer between 1 to 50, and still more preferably an integer between 1 to 30.

y1 + y2 is preferably an integer between 5 to 2,000, more preferably an integer between 7 to 1,000, still more preferably an integer between 10 to 50, and particularly preferably an integer between 15 to 30.

As a combination of $R^{b1}$ to $R^{b3}$, a combination of a hydrogen atom or an alkyl group having 1 to 4 carbon atoms as $R^{b1}$, an alkyl group having 1 to 4 carbon atoms as $R^{b2}$, and a hydrogen atom as $R^{b3}$ is preferable and a combination of an alkyl group having 1 to 4 carbon atoms as $R^{b1}$, an alkyl group having 1 to 4 carbon atoms as $R^{b2}$, and a hydrogen atom as $R^{b3}$ is more preferable.

In these preferred combinations, the content of a hydrosilyl group represented by y2 / (y1 + y2) is preferably greater than 0.1 and less than 0.6 and more preferably greater than 0.1 and less than 0.4.

Examples of the polyorganosiloxane (B) with a linear structure include HMS-064 (MeHSio: 5 to 7 mol%), HMS-082 (MeHSio: 7 to 8 mol%), HMS-301 (MeHSio: 25 to 30 mol%), HMS-501 (MeHSio: 50 to 55 mol%) which are all methylhydrosiloxane-dimethylsiloxane copolymers (trimethylsiloxane terminated) manufactured by GELEST, INC..

Here, mol% of MeHSiO is synonymous with a value obtained by multiplying y2 / (y1 + y2) by 100 in the above-described preferred combinations of $R^{b1}$ to $R^{b3}$.

It is preferable that both the linear structure and the branched structure have no vinyl group from the viewpoint of

preventing the progress of a crosslinking reaction within a molecule. Among these, it is preferable that the branched structure has no vinyl group.

The polyorganosiloxane (B) which has a branched structure and two or more Si-H groups in a molecular chain has a branched structure and two or more hydrosilyl groups (Si-H groups).

The specific gravity is preferably 0.9 to 0.95.

The polyorganosiloxane (B) with a branched structure is preferably polyorganosiloxane represented by the following Average Composition Formula (b).

Average Composition Formula (b): $[H_a(R^{b6})_{3-a}SiO_{1/2}]_{y3}[SiO_{4/2}]_{y4}$

Here, $R^{b6}$ represents an alkyl group, a cycloalkyl group, an alkenyl group, or an aryl group, a represents 0.1 to 3, and y3 and y4 each independently represent an integer of 1 or more.

An alkyl group, a cycloalkyl group, an alkenyl group, and an aryl group in $R^{b6}$ are synonymous with an alkyl group, a cycloalkyl group, an alkenyl group, and an aryl group in $R^{a2}$ and $R^{a3}$, and preferred ranges thereof are also the same as each other.

a is preferably 1.

The content of a hydrosilyl group represented by a/3 is preferably greater than 0.1 and less than 0.6 and more preferably greater than 0.1 and less than 0.4.

In contrast, in a case of representing the polyorganosiloxane (B) with a branched structure, polyorganosiloxane in which $-O-Si(CH_3)_2(H)$ is bonded to a Si atom constituting a main chain is preferable and polyorganosiloxane having a structure represented by the following General Formula (Bb) is more preferable.

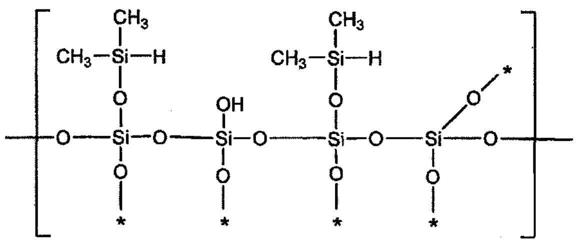 (Bb)

In General Formula (Bb), * means a bond with at least a Si atom of siloxane.

Examples of the polyorganosiloxane (B) with a branched structure include HQM-107 (trade name of Hydride Q Resin manufactured by GELEST, INC.) and HDP-111 (trade name of POLYPHENYL-(DIMETHYLHYDROXY)SILOXANE (HYDRIDE TERMINATED), $[(HM_{e2}SiO)(C_6H_3Si)O]$: 99 to 100 mol% manufactured by GELEST, INC.).

The polyorganosiloxane (B) having two or more Si-H groups in a molecular chain used in the present invention may be used singly, or two or more thereof may be used in combination. In addition, the polyorganosiloxane (B) with a linear structure and the polyorganosiloxane (B) with a branched structure may be used in combination.

<Inorganic Compound Particles (C)>

The average primary particle diameter of the inorganic compound particles (C) used in the present invention is less than 25 nm and is selected from the group consisting of magnesium oxide, titanium oxide, iron oxide, zinc oxide, zirconium oxide, barium oxide, tin oxide, and ytterbium oxide.

By adding inorganic compound particles to a silicone resin, the acoustic attenuation is increased in accordance with the increase in the amount of inorganic compound particles added while effects of improving the acoustic impedance, the hardness, and the mechanical strength of the silicone resin can be obtained.

However, in the present invention, by setting the average primary particle diameter of the inorganic compound particles (C) to be small being less than 25 nm, it is considered that the increase in acoustic attenuation is suppressed, and therefore, the tear strength of a silicone resin can be improved.

That is, it is considered that cracking of a silicone resin caused by mechanical stress is suppressed due to inorganic compound particles (C) functioning as stoppers. Particularly, since the distance between the particles becomes short in a case where the average primary particle diameter is small, the inorganic compound particles more favorably exhibit functions of stoppers. Therefore, it is assumed that the tear strength of the silicone resin is significantly improved.

The average primary particle diameter of the inorganic compound particles (C) used in the present invention is less than 25 nm, preferably greater than 3 nm and less than 25 nm, more preferably greater than 3 nm and less than 20 nm, and still more preferably greater than 3 nm and less than 15 nm from the viewpoint of suppressing the increase in acoustic attenuation of the silicone resin and improving the tear strength. As the average primary particle diameter is within the

above-described range and smaller, the tear strength becomes higher and excellent acoustic sensitivity is exhibited, which is preferable.

The average primary particle diameter is disclosed in a catalog of a manufacturer of inorganic compound particles. However, for inorganic compound particles which have not been disclosed in the catalog or newly manufactured, it is possible to obtain the average primary particle diameter by averaging the particle diameters measured using a transmission electron microscope (transmission electron microscopy: TEM). That is, the minor axis and the major axis of a particle of an electron micrograph photographed using TEM were measured and an average value thereof was obtained as the particle diameter of a particle. In the present specification, the particle diameters of 300 or more particles are averaged and the averaged particle diameter is obtained as the average primary particle diameter.

In addition, in a case where surface treatment to be described below is performed on the inorganic compound particles (C), the average primary particle diameter means an average primary particle diameter in a state in which the surface treatment has been performed.

In addition, the specific gravity of the inorganic compound particles (C) used in the present invention is preferably 2.5 to 10.0, and the lower limit value thereof is preferably greater than or equal to 4.0 and more preferably greater than or equal to 5.0.

Specifically, inorganic compound particles selected from the group consisting of titanium oxide, iron oxide, zinc oxide, zirconium oxide, barium oxide, tin oxide, and ytterbium oxide are preferable and inorganic compound particles selected from the group consisting of iron oxide, zinc oxide, zirconium oxide, barium oxide, tin oxide, and ytterbium oxide are more preferable.

The inorganic compound particles (C) may be used singly, or two or more thereof may be used in combination.

The specific surface area of the inorganic compound particles (C) used in the present invention is preferably 50 to 400 $m^2/g$ and more preferably 100 to 400 $m^2/g$ from the viewpoint of improving the hardness or the mechanical strength of an obtained silicone resin.

As the inorganic compound particles (C) used in the present invention, inorganic compound particles of which the surfaces have been treated using a silane compound are required.

By treating the surfaces of inorganic compound particles using a silane compound, interaction with the silicone resin becomes stronger and affinity to the silicone resin becomes higher. Therefore, it is considered that it is possible to finely disperse inorganic compound particles with a small average primary particle diameter. For this reason, the inorganic compound particles (C) more favorably exhibit functions of stoppers in a case where mechanical stress is applied, and therefore, it is considered that the hardness and the mechanical strength of the silicone resin are improved.

A usual technique may be used as a technique of the surface treatment. Examples of the technique of the surface treatment using a silane compound include a technique of performing surface treatment using a silane coupling agent.

Silane Coupling Agent

A silane coupling agent having a hydrolyzable group is preferable as a silane coupling agent from the viewpoint of improving the hardness or the mechanical strength of a silicone resin. Surface modification of inorganic compound particles is performed such that a hydrolyzable group in a silane coupling agent becomes a hydroxyl group after being hydrolyzed using water and this hydroxyl group is subjected to a dehydration and condensation reaction with a hydroxyl group on the surfaces of the inorganic compound particles, thereby improving the hardness or the mechanical strength of an obtained silicone resin. Examples of the hydrolyzable group include an alkoxy group, an acyloxy group, and a halogen atom.

In a case where the surfaces of inorganic compound particles are hydrophobically modified, affinity between the inorganic compound particles (C), the polyorganosiloxane (A), and the polyorganosiloxane (B) becomes favorable, and therefore, the hardness and the mechanical strength of an obtained silicone resin is improved, which is preferable.

Examples of a silane coupling agent having a hydrophobic group as a functional group include alkoxysilanes such as methyltrimethoxysilane, dimethyldimethoxysilane, phenyltrimethoxysilane, methyltriethoxysilane, dimethyldiethoxysilane, phenyltriethoxysilane, n-propyltrimethoxysilane, n-propyltriethoxysilane, hexyltrimethoxysilane, hexyltriethoxysilane, and decyltrimethoxysilane; chlorosilanes such as methyltrichlorosilane, dimethyldichlorosilane, trimethylchlorosilane, and phenyltrichlorosilane; and hexamethyldisilazane (HMDS).

In addition, examples of a silane coupling agent having a vinyl group as a functional group include alkoxysilanes such as methacryloxypropyltriethoxysilane, methacryloxypropyltrimethoxysilane, methacryloxypropylmethyldiethoxysilane, methacryloxypropylmethyldimethoxysilane, vinyltriethoxysilane, vinyltrimethoxysilane, and vinylmethyldimethoxysilane; chlorosilane such as vinyltrichlorosilane and vinylmethyldichlorosilane; and divinyltetramethyldisilazane.

Inorganic compound particles treated using a trialkylsilating agent are preferable and inorganic compound particles treated using a trimethylsilating agent are more preferable as the inorganic compound particles (C) subjected to surface treatment using a silane coupling agent.

Examples of the silane compound include the above-described silane coupling agents and a silane coupling

agent in which a functional group in a silane coupling agent is substituted with an alkyl group.

In addition, examples of the trimethylsilating agent include trimethylchlorosilane and hexamethyldisilazane (HMDS) described in the above-described silane coupling agent or trimethylmethoxysilane which is a silane coupling agent in which a functional group is substituted with an alkyl group.

An example of a commercially available silane coupling agent includes hexamethyldisilazane (HMDS) (trade name: HEXAMETHYLDISILAZANE (SIH6110.1), manufactured by GELEST, INC.).

A hydroxyl group existing on the surfaces of inorganic compound particles is covered with a trimethylsilyl group through a reaction with hexamethyldisilazane (HMDS) and the surfaces of the inorganic compound particles are hydrophobically modified.

In general, the vinyl group possessed by the polyorganosiloxane (A) and the Si-H group possessed by the polyorganosiloxane (B) stoichiometrically react with each other in a ratio of 1:1.

However, in the present invention, the average primary particle diameter of the inorganic compound particles (C) is small and the space between the polyorganosiloxane (A) and the polyorganosiloxane (B) is densely filled with the inorganic compound particles (C). Therefore, the movement of molecular chains of the polyorganosiloxane (A) and the polyorganosiloxane (B) is restricted.

Accordingly, the equivalent of the Si-H groups possessed by the polyorganosiloxane (B) to the vinyl group possessed by the polyorganosiloxane (A) for a reaction between all the vinyl groups with the Si-H groups is preferably vinyl group : Si-H group = 1 : 1.1 to 1 : 8 and more preferably 1 : 1.2 to 1 :5.

<Other Components>

In the composition for an acoustic wave probe of the present invention, it is possible to appropriately formulate a platinum catalyst for an addition polymerization reaction, a vulcanization retardant, a solvent, a dispersant, a pigment, a dye, an antistatic agent, an antioxidant, a flame retardant, a thermal conductivity enhancer in addition to the polyorganosiloxane (A) having a vinyl group, a polyorganosiloxane (B) having two or more Si-H groups in a molecular chain, and the inorganic compound particles (C).

-Catalyst-

Examples of the catalyst include platinum or a platinum-containing compound (hereinafter, also referred to as a platinum compound). Any platinum or platinum compound can be used.

Specific examples thereof include platinum black; a catalyst in which platinum is carried on an inorganic compound, carbon black, or the like; platinum chloride or an alcohol solution of platinum chloride; a complex salt of platinum chloride and olefin; and a complex salt of platinum chloride and vinyl siloxane. The catalyst may be used singly, or two or more thereof may be used in combination.

The content of a catalyst can appropriately be set within a range of the amount of catalyst.

The catalyst is necessary in the hydrosilylation reaction in which the Si-H groups of the polyorganosiloxane (B) are added to the vinyl group of the polyorganosiloxane (A). The polyorganosiloxane (A) is cross-linked by the polyorganosiloxane (B) through an addition vulcanization reaction due to hydrosilylation to form a silicone resin.

Here, the catalyst may be contained in the composition for an acoustic wave probe of the present invention or may be brought into contact with the composition for an acoustic wave probe without being contained in the composition for an acoustic wave probe. The latter case is preferable.

An example of a commercially available platinum catalyst includes a platinum compound (trade name: PLATINUM CYCLOVINYLMETHYLSILOXANE COMPLEX IN CYCLIC METHYLVINYLSILOXANES (SIP6832.2), 2 mass% of Pt concentration, manufactured by GELEST, INC.).

In a case where a catalyst is contained in the composition for an acoustic wave probe of the present invention, the content of the catalyst with respect to 100 parts by mass of a polysiloxane mixture is preferably 0.00001 to 0.05 parts by mass, more preferably 0.00001 to 0.01 parts by mass, still more preferably 0.00002 to 0.01 parts by mass, and particularly preferably 0.00005 to 0.005 parts by mass from the viewpoint of reactivity.

In addition, it is possible to control the vulcanization temperature by selecting an appropriate platinum catalyst. For example, platinum-vinyldisiloxane is used for room temperature vulcanization (RTV) at lower than or equal to 50°C and platinum-cyclic vinylsiloxane is used for high temperature vulcanization (HTV) at higher than or equal to 130°C.

-Vulcanization Retardant-

In the present invention, it is possible to appropriately use a vulcanization retardant for a vulcanization reaction. The vulcanization retardant is used for retarding an addition vulcanization reaction performed using a platinum catalyst. Examples thereof include a vinylmethylsiloxane homopolymer with a low molecular weight (trade name: VMS-005,

manufactured by GELEST, INC.).

The vulcanization rate, that is, the operation time can be adjusted in accordance with the content of the vulcanization retardant.

<Method for Producing Composition for Acoustic Wave Probe and Silicone Resin for Acoustic Wave Probe>

The composition for an acoustic wave probe of the present invention can be produced through an arbitrary method.

For example, the composition for an acoustic wave probe can be obtained by kneading components constituting the composition for an acoustic wave probe using a kneader, a pressure kneader, a Banbury mixer (continuous kneader), and a kneading device with two rolls. The order of mixing the components is not particularly limited.

It is preferable to first make a polyorganosiloxane mixture in which the inorganic compound particles (C) are dispersed in the polyorganosiloxane (A) having a vinyl group and the polyorganosiloxane (B) having two or more Si-H groups in a molecular chain, from the viewpoint of obtaining a homogeneous composition. Thereafter, it is possible to produce a composition for an acoustic wave probe after adding a catalyst to the polyorganosiloxane mixture, in which the inorganic compound particles (C) are dispersed, and performing defoamation under reduced pressure.

It is possible to obtain a silicone resin for an acoustic wave probe of the present invention by vulcanizing the composition for an acoustic wave probe of the present invention which has been obtained in this manner. Specifically, it is possible to obtain a silicone resin for an acoustic wave probe by, for example, thermally vulcanizing the composition for an acoustic wave probe for 5 minutes to 500 minutes at 20°C to 200°C.

<Mechanical Strength and Acoustic Characteristics of Silicone Resin>

The silicone resin for an acoustic wave probe of the present invention is obtained by vulcanizing the composition for an acoustic wave probe of the present invention.

Hereinafter, the mechanical strength and acoustic characteristics of a silicone resin will be described in detail.

Here, ultrasonic characteristics among acoustic characteristics will be described. However, the acoustic characteristics are not limited to ultrasonic characteristics, and relates to acoustic characteristics at an appropriate frequency which is selected in accordance with a test object, measurement conditions, or the like.

[Hardness]

The type A durometer hardness of a silicone resin sheet with a thickness of 2 mm is measured using a rubber hardness meter (for example, trade name "RH-201A" manufactured by Excel co., Ltd.) in compliance with JIS K6253-3 (2012).

The hardness is preferably greater than or equal to 15 and more preferably greater than or equal to 25 from the viewpoint of preventing modification while using the sheet in an embedded form as a part of an acoustic wave probe. A practical upper limit value is less than or equal to 80.

[Tensile Test]

A dumbbell-like test piece of a silicone resin sheet with a thickness of 1 mm is manufactured and the tensile strength at break and the tensile elongation at break (elongation) are measured in compliance with JIS K6251 (2010).

The tensile strength at break is preferably greater than or equal to 1.2 MPa and the tensile elongation at break is preferably greater than or equal to 500%. A practical upper limit value of the tensile strength at break is less than or equal to 10 MPa and a practical upper limit value of the tensile elongation at break is less than or equal to 1,500%.

[Tear Strength Test]

A trouser-type test piece of a silicone resin sheet with a thickness of 2 mm is manufactured and the tear strength is measured in compliance with JIS K6252 (2007).

The tear strength is preferably greater than or equal to 15 N/cm, more preferably greater than or equal to 20 N/cm, and still more preferably greater than or equal to 30 N/cm. A practical upper limit value is less than or equal to 100 N/cm.

[Abrasion Resistance Test]

A Taber abrasion test is performed on a silicone resin sheet with a thickness of 2 mm and mass decrement is measured in compliance with JIS K6264-2 (2005). The measurement is performed under the conditions of a polishing

wheel of H22, a load of 9.8 N, and a test rotation speed of 1,000 rotations. The mass decrement less than 30 mg is set to "A", the mass decrement greater than or equal to 30 mg and less than 50 mg is set to "B", the mass decrement greater than or equal to 50 mg and less than 70 mg is set to "C", and the mass decrement greater than 70 mg is set to "D".

Here, "A" and "B" of the evaluation indicate that the sheet has considerably excellent abrasion resistance, "C" indicates that the sheet is in a usable state, and "D" indicates that the sheet is in an unusable state.

[Acoustic Impedance]

The density of a silicone resin sheet with a thickness of 2 mm at 25°C is measured using an electronic gravimeter (for example, "SD-200L" manufactured by ALFA MIRAGE) in accordance with a density measurement method of a method A (underwater substitution method) disclosed in JIS K7112 (1999). The acoustic velocity of an acoustic wave is measured at 25°C using a sing-around type acoustic velocity measurement apparatus (for example, "UVM-2 type" manufactured by Ultrasonic Engineering Co., LTd.) in compliance with JIS Z2353 (2003) and acoustic impedance is obtained from a sum of the density and the acoustic velocity which have been measured.

[Acoustic (Ultrasonic) Attenuation and Sensitivity]

A sinusoidal signal (a wave) of 5 MHz which has been output from an ultrasonic oscillator (for example, a function generator with a trade name of "FG-350" manufactured by IWATSU ELECTRIC CO.,LTD.) is input into an ultrasound probe (manufactured by, for example, JAPAN PROBE), and an ultrasonic pulse wave with a center frequency of 5 MHz is generated in water from the ultrasound probe. The magnitude of the amplitude before and after the generated ultrasonic wave passes through a silicone resin sheet with a thickness of 2 mm is measured in a water temperature environment of 25°C using an ultrasonic receiver (for example, an oscilloscope with a trade name of "VP-5204A" manufactured by Matsushita Electric Industrial Co., LTd.). The acoustic (ultrasonic) attenuation of each sheet is compared with each other by comparing the acoustic (ultrasonic) sensitivities of each sheet.

The acoustic (ultrasonic) sensitivity is regarded as a numerical value given by the following calculation equation.

In the following calculation equation, Vin represents a voltage peak value of a wave with a half-width of less than or equal to 50 nsec which has been input using the ultrasonic oscillator. Vs represents a voltage value obtained in a case where the generated acoustic (ultrasonic) wave passes through the sheets and the ultrasonic oscillator receives the acoustic (ultrasonic) wave which has been reflected from a surface facing the sheets.

$$\text{Acoustic (ultrasonic) sensitivity} = 20 \times \text{Log}\,(\text{Vs} / \text{Vin})$$

In an evaluation system in the present invention, the acoustic (ultrasonic) sensitivity is preferably greater than or equal to -72 dB and more preferably greater than or equal to -71 dB.

The composition for an acoustic wave probe of the present invention is useful for a medical instrument and can preferably be used in, for example, an acoustic wave probe or an acoustic wave measurement apparatus. The acoustic wave measurement apparatus of the present invention is not limited to an ultrasound diagnostic apparatus or a photoacoustic wave measurement apparatus, and is referred to as an apparatus that receives an acoustic wave which has been reflected or generated from an object and displays the received acoustic wave as an image or a signal strength.

Particularly, the composition for an acoustic wave probe of the present invention can suitably be used in: a material of an acoustic matching layer which is provided in an acoustic lens of an ultrasound diagnostic apparatus or between a piezoelectric element and the acoustic lens and plays a role of matching acoustic impedance between the piezoelectric element and the acoustic lens; a material of an acoustic lens in a photoacoustic wave measurement apparatus or an ultrasound endoscope; and a material or the like of an acoustic lens in an ultrasound probe including capacitive micromachined ultrasonic transducers (cMUT) as an ultrasonic transducer array.

Specifically, the silicone resin for an acoustic wave probe of the present invention is preferably applied to, for example, an ultrasound diagnostic apparatus disclosed in JP2005-253751A and JP2003-169802A or an acoustic wave measurement apparatus such as a photoacoustic wave measurement apparatus disclosed in JP2013-202050A, JP2013-188465A, JP2013-180330A, JP2013-158435A, JP2013-154139A, or the like.

<<Acoustic Wave Probe>>

A configuration of an acoustic wave probe of the present invention will be described below in more detail based on a configuration of an ultrasound probe in an ultrasound diagnostic apparatus which is described in Fig. 1. The ultrasound probe is a probe which particularly uses an ultrasonic wave as an acoustic wave in an acoustic wave probe. For this reason,

the basic configuration of the ultrasound probe can be applied to the acoustic wave probe as it is.

-Ultrasound probe-

An ultrasound probe 10 is a main component of the ultrasound diagnostic apparatus and has a function of generating an ultrasonic wave and transmitting and receiving an ultrasonic beam. The configuration of the ultrasound probe 10 is provided in the order of an acoustic lens 1, an acoustic matching layer 2, a piezoelectric element layer 3, and a backing material 4 from a distal end (the surface coming into contact with a living body which is a test object) as shown in Fig. 1. In recent years, an ultrasound probe having a layered structure in which an ultrasonic transducer (piezoelectric element) for transmission and an ultrasonic transducer (piezoelectric element) for reception are formed of materials different from each other has been proposed in order to receive high-order harmonics.

<Piezoelectric Element Layer>

The piezoelectric element layer 3 is a portion which generates an ultrasonic wave and in which an electrode is attached to both sides of a piezoelectric element. In a case where voltage is applied to the electrode, the piezoelectric element layer generates an ultrasonic wave through repeated contraction and expansion of the piezoelectric element and through vibration.

Inorganic piezoelectric bodies of so-called ceramics obtained by polarizing crystals, single crystals such as $LiNbO_3$, $LiTaO_3$, and $KNbO_3$, thin films of ZnO and AlN, $Pb(Zr,Ti)O_3$-based sintered body, and the like are widely used as the material constituting a piezoelectric element. In general, piezoelectric ceramics such as lead zirconate titanate (PZT) with a good conversion efficiency are used.

In addition, sensitivity having a wider band width is required for a piezoelectric element detecting a reception wave on a high frequency side. For this reason, an organic piezoelectric body has been used in which an organic polymer material such as polyvinylidene fluoride (PVDF) is used as the piezoelectric element being suitable for a high frequency or a wide band.

Furthermore, cMUT using micro electro mechanical systems (MEMS) technology in which an array structure, which shows excellent short pulse characteristics, excellent broadband characteristics, and excellent mass productivity and has less characteristic variations, is obtained is disclosed in JP2011-071842A or the like.

In the present invention, it is possible to preferably use any piezoelectric element material.

<Backing Material>

The backing material 4 is provided on a rear surface of the piezoelectric element layer 3 and contributes to the improvement in distance resolution in an ultrasonic diagnostic image by shortening the pulse width of an ultrasonic wave through the suppression of excess vibration.

<Acoustic Matching Layer>

The acoustic matching layer 2 is provided in order to reduce the difference in acoustic impedance between the piezoelectric element layer 3 and a test object and to efficiently transmit and receive an ultrasonic wave.

A composition for an ultrasound probe of the present invention can preferably be used as a material for the acoustic matching layer since the difference in acoustic impedance ($1.4 \times 10^6$ to $1.7 \times 10^6$ $kg/m^2/sec$) between the piezoelectric element layer and a living body is small. The acoustic matching layer of the present invention preferably contains 10 mass% or more of a silicone resin for an acoustic wave probe obtained by subjecting the composition for an acoustic wave probe of the present invention to a vulcanization reaction.

<Acoustic Lens>

The acoustic lens 1 is provided in order to improve resolution by making an ultrasonic wave converge in a slice direction using refraction. In addition, it is necessary for the acoustic lens to achieve matching of an ultrasonic wave with acoustic impedance ($1.4 \times 10^6$ to $1.7 \times 10^6$ $kg/m^2/sec$ in a case of a human body) of a living body which is a test object after being closely attached to the living body and to reduce ultrasonic attenuation of the acoustic lens 1 itself.

That is, sensitivity of transmission and reception of an ultrasonic wave is improved using a material, of which the acoustic velocity is sufficiently lower than that of a human body, the ultrasonic attenuation is low, and the acoustic impedance is close to a value of the skin of a human body, as the material of the acoustic lens 1.

The composition for an acoustic wave probe which is the composition for an ultrasound probe of the present invention can also preferably be used as a material of the acoustic lens.

The operation of the ultrasound probe 10 having such a configuration will be described. The piezoelectric element layer 3 is resonated after applying voltage to the electrodes provided on both sides of a piezoelectric element, and an ultrasonic signal is transmitted to a test object from the acoustic lens. During reception of the ultrasonic signal, the piezoelectric element layer 3 is vibrated using the signal (echo signal) reflected from the test object and this vibration is electrically converted into a signal to obtain an image.

Particularly, a remarkable effect of improving the sensitivity can be checked from a transmission frequency of an ultrasonic wave of greater than or equal to about 5 MHz using the acoustic lens obtained from the composition for an ultrasound probe of the present invention as a general medical ultrasonic transducer. Particularly, a remarkable effect of improving the sensitivity can particularly be expected from a transmission frequency of an ultrasonic wave of greater than or equal to 10 MHz.

Hereinafter, an apparatus in which the acoustic lens obtained from the composition for an ultrasound probe of the present invention exhibits a function particularly regarding conventional problems will be described in detail.

The composition for an ultrasound probe of the present invention exhibits an excellent effect even with respect to other apparatuses disclosed below.

-Ultrasound probe including Capacitive Micromachined Ultrasonic Transducer (cMUT)-

In a case where cMUT apparatuses disclosed in JP2006-157320A, JP2011-71842A, and the like are used in an ultrasonic diagnostic transducer array, the sensitivity thereof generally becomes low compared to a transducer in which usual piezoelectric ceramics (PZT) is used.

However, it is possible to make up for deficient sensitivity of cMUT using the acoustic lens obtained from the composition for an acoustic wave probe of the present invention. Accordingly, it is possible to make the sensitivity of cMUT to performance of a conventional transducer.

The cMUT apparatus is manufactured through MEMS technology. Therefore, it is possible to provide an inexpensive ultrasound probe, of which mass productivity is higher than that of a piezoelectric ceramics probe, to the market.

-Photoacoustic Wave Measurement apparatus Using Photoultrasonic Imaging-

Photoultrasonic imaging (photo acoustic imaging: PAI) disclosed in JP2013-158435A or the like displays a signal strength of an ultrasonic wave or an image obtained by imaging the ultrasonic wave generated when human tissue is adiabatically expanded using light (magnetic wave) with which the interior of a human body is irradiated.

Here, the amount of an acoustic pressure of an ultrasonic wave generated through light irradiation is minute, and therefore, there is a problem in that it is difficult to observe deeper regions of a human body.

However, it is possible to exhibit an effect effective for the problem using the acoustic lens obtained from the composition for an acoustic wave probe of the present invention.

-Ultrasound endoscope-

In an ultrasonic wave in an ultrasound endoscope disclosed in JP2008-311700A or the like, a signal line cable is structurally long compared to that of a transducer for a body surface, and therefore, there is a problem of improving the sensitivity of the transducer caused by loss of the cable. Regarding this problem, it is said that there are no effective means for improving the sensitivity due to the following reasons.

First, in a case of an ultrasound diagnostic apparatus for a body surface, it is possible to install an amplifier circuit, an AD conversion IC, or the like at a distal end of the transducer. In contrast, the ultrasound endoscope is inserted into a body. Therefore, the installation space within the transducer is narrow, and thus, it is difficult to install an amplifier circuit, an AD conversion IC, or the like at a distal end of the transducer.

Secondly, it is difficult to apply a piezoelectric single crystal employed in the transducer in the ultrasound diagnostic apparatus for a body surface onto a transducer with an ultrasonic transmission frequency of greater than or equal to 7 to 8 MHz due to physical properties and processing suitability. However, an ultrasonic wave for an endoscope is generally a probe having an ultrasonic transmission frequency of greater than or equal to 7 to 8 MHz, and therefore, it is also difficult to improve thesensitivity due to piezoelectric single crystal material.

However, it is possible to improve the sensitivity of the ultrasonic transducer for an endoscope using the acoustic lens obtained from the composition for an acoustic wave probe of the present invention.

In addition, even in a case of using the same ultrasonic transmission frequency (for example, 10 MHz), the efficacy is particularly exhibited in a case of using the acoustic lens obtained from the composition for an acoustic wave probe of the present invention in the ultrasonic transducer for an endoscope.

[Examples]

The present invention will be described in more detail based on Examples in which an ultrasonic wave is used as an acoustic wave. The present invention is not limited to the ultrasonic wave, and any acoustic wave of an audible frequency may be used as long as an appropriate frequency is selected in accordance with a test object, measurement conditions, and the like.

[Example 1]

68 parts by mass of vinyl terminated polydimethylsiloxane (trade name of "DMS-V42" with a mass average molecular weight of 72,000, manufactured by GELEST, INC.), 2 parts by mass of a methylhydrosiloxane-dimethylsiloxane copolymer (trade name of "HMS-301" with a mass average molecular weight of 2,000 and a proportion of methylhydrosiloxane of 27 mol%, manufactured by GELEST, INC.), and 30 parts by mass of magnesium oxide (specific gravity of 3.6, average primary particle diameter of 19 nm, and surface treatment using hexamethyldisilazane (HMDS)) were kneaded using a kneader for 2 hours to make a homogeneous paste. 0.05 parts by mass of a platinum catalyst solution (a trade name of "SIP6832.2" with 2 mass% of Pt concentration, manufactured by GELEST, INC.) was added to and mixed with the paste. Then, the mixture was subjected to defoamation under reduced pressure, was placed in metal molds of 150 mm x 150 mm, and was subjected to heat treatment at 60°C for 3 hours to obtain silicone resin sheets each having a thickness of 1 mm and 2 mm.

[Example 2]

Predetermined silicone resin sheets were obtained after performing a treatment similarly to Example 1 except that 30 parts by mass of titanium oxide (specific gravity of 4.2, average primary particle diameter of 14 nm, and surface treatment using hexamethyldisilazane (HMDS)) was used as inorganic compound particles.

[Example 3]

Predetermined silicone resin sheets were obtained after performing a treatment similarly to Example 1 except that 30 parts by mass of iron oxide (specific gravity of 5.2, average primary particle diameter of 21 nm, and surface treatment using hexamethyldisilazane (HMDS)) was used as inorganic compound particles.

[Example 4]

Predetermined silicone resin sheets were obtained similarly to Example 1 such that 68 parts by mass of vinyl terminated polydimethylsiloxane (trade name of "DMS-V42" with a mass average molecular weight of 72,000, manufactured by GELEST, INC.), 2 parts by mass of a methylhydrosiloxane-dimethylsiloxane copolymer (trade name of "HMS-301" with a mass average molecular weight of 2,000 and a proportion of methylhydrosiloxane of 27 mol%, manufactured by GELEST, INC.), and 30 parts by mass of zinc oxide (specific gravity of 5.6, average primary particle diameter of 11 nm, and surface treatment using hexamethyldisilazane (HMDS)) were mixed with each other similarly to Example 1 and the mixture was thermally vulcanized using a platinum catalyst.

[Example 5]

Predetermined silicone resin sheets were obtained after performing a treatment similarly to Example 1 except that 30 parts by mass of zirconium oxide (specific gravity of 5.9, average primary particle diameter of 11 nm, and surface treatment using hexamethyldisilazane (HMDS)) was used as inorganic compound particles.

[Example 6]

Predetermined silicone resin sheets were obtained after performing a treatment similarly to Example 1 except that 30 parts by mass of barium oxide (specific gravity of 6.7, average primary particle diameter of 24 nm, and surface treatment using hexamethyldisilazane (HMDS)) was used as inorganic compound particles.

[Example 7]

Predetermined silicone resin sheets were obtained after performing a treatment similarly to Example 1 except that 30 parts by mass of tin oxide (specific gravity of 7.0, average primary particle diameter of 22 nm, and surface treatment

using hexamethyldisilazane (HMDS)) was used as inorganic compound particles.

[Example 8]

Predetermined silicone resin sheets were obtained after performing a treatment similarly to Example 1 except that 30 parts by mass of ytterbium oxide (specific gravity of 9.2, average primary particle diameter of 20 nm, and surface treatment using hexamethyldisilazane (HMDS)) was used as inorganic compound particles.

[Example 9] (not according to the invention)

Predetermined silicone resin sheets were obtained after performing a treatment similarly to Example 4 except that 30 parts by mass of zinc oxide (specific gravity of 5.6, average primary particle diameter of 11 nm, and no surface treatment) was used as inorganic compound particles.

[Example 10]

Predetermined silicone resin sheets were obtained after performing a treatment similarly to Example 4 except that 65 parts by mass of vinyl terminated polydimethylsiloxane (trade name of "DMS-V31" with a mass average molecular weight of 28,000, manufactured by GELEST, INC.) was used as polyorganosiloxane having a vinyl group and 5 parts by mass of methylhydrosiloxane-dimethylsiloxane copolymer (trade name of "HMS-301" with a mass average molecular weight of 2,000 and a proportion of methylhydrosiloxane of 27 mol%, manufactured by GELEST, INC.) was used as polyorganosiloxane having an Si-H group.

[Example 11]

Predetermined silicone resin sheets were obtained after performing a treatment similarly to Example 4 except that 67 parts by mass of vinyl terminated polydimethylsiloxane (trade name of "DMS-V35" with a mass average molecular weight of 49,500, manufactured by GELEST, INC.) was used as polyorganosiloxane having a vinyl group and 3 parts by mass of methylhydrosiloxane-dimethylsiloxane copolymer (trade name of "HMS-301" with a mass average molecular weight of 2,000 and a proportion of methylhydrosiloxane of 27 mol%, manufactured by GELEST, INC.) was used as polyorganosiloxane having an Si-H group.

[Example 12]

Predetermined silicone resin sheets were obtained after performing a treatment similarly to Example 4 except that 69 parts by mass of vinyl terminated polydimethylsiloxane (trade name of "DMS-V46" with a mass average molecular weight of 117,000, manufactured by GELEST, INC.) was used as polyorganosiloxane having a vinyl group and 1 parts by mass of methylhydrosiloxane-dimethylsiloxane copolymer (trade name of "HMS-301" with a mass average molecular weight of 2,000 and a proportion of methylhydrosiloxane of 27 mol%, manufactured by GELEST, INC.) was used as polyorganosiloxane having an Si-H group.

[Example 13]

Predetermined silicone resin sheets were obtained after performing a treatment similarly to Example 4 except that 69 parts by mass of vinyl terminated polydimethylsiloxane (trade name of "DMS-V52" with a mass average molecular weight of 155,000, manufactured by GELEST, INC.) was used as polyorganosiloxane having a vinyl group and 1 parts by mass of methylhydrosiloxane-dimethylsiloxane copolymer (trade name of "HMS-301" with a mass average molecular weight of 2,000 and a proportion of methylhydrosiloxane of 27 mol%, manufactured by GELEST, INC.) was used as polyorganosiloxane having an Si-H group.

[Example 14]

Predetermined silicone resin sheets were obtained after performing a treatment similarly to Example 4 except that 78 parts by mass of vinyl terminated polydimethylsiloxane (trade name of "DMS-V42" with a mass average molecular weight of 72,000, manufactured by GELEST, INC.) was used as polyorganosiloxane having a vinyl group, 2 parts by mass of methylhydrosiloxane-dimethylsiloxane copolymer (trade name of "HMS-301" with a mass average molecular weight of 2,000 and a proportion of methylhydrosiloxane of 27 mol%, manufactured by GELEST, INC.) was used as polyorganosiloxane having an Si-H group, and 20 parts by mass of zinc oxide (specific gravity of 5.6, average primary particle diameter

of 11 nm, and surface treatment using hexamethyldisilazane (HMDS)) was used as inorganic compound particles.

[Example 15]

Predetermined silicone resin sheets were obtained after performing a treatment similarly to Example 4 except that 59 parts by mass of vinyl terminated polydimethylsiloxane (trade name of "DMS-V42" with a mass average molecular weight of 72,000, manufactured by GELEST, INC.) was used as polyorganosiloxane having a vinyl group, 1 part by mass of methylhydrosiloxane-dimethylsiloxane copolymer (trade name of "HMS-301" with a mass average molecular weight of 2,000 and a proportion of methylhydrosiloxane of 27 mol%, manufactured by GELEST, INC.) was used as polyorganosiloxane having an Si-H group, and 40 parts by mass of zinc oxide (specific gravity of 5.6, average primary particle diameter of 11 nm, and surface treatment using hexamethyldisilazane (HMDS)) was used as inorganic compound particles.

[Example 16]

Predetermined silicone resin sheets were obtained after performing a treatment similarly to Example 8 except that 68 parts by mass of a vinyl terminated diphenylsiloxane-dimethylsiloxane copolymer (trade name of "PDV-0535" with a mass average molecular weight of 47,500 and an amount of diphenylsiloxane of 5 mol%, manufactured by GELEST, INC.) was used as polyorganosiloxane having a vinyl group.

[Example 17]

Predetermined silicone resin sheets were obtained after performing a treatment similarly to Example 8 except that 68 parts by mass of a vinyl terminated diphenylsiloxane-dimethylsiloxane copolymer (trade name of "PDV-1635" with a mass average molecular weight of 35,300 and an amount of diphenylsiloxane of 16 mol%, manufactured by GELEST, INC.) was used as polyorganosiloxane having a vinyl group.

[Comparative Example 1]

Predetermined silicone resin sheets were obtained after performing a treatment similarly to Example 1 except that 30 parts by mass of magnesium oxide (specific gravity of 3.6, average primary particle diameter of 60 nm, and surface treatment using hexamethyldisilazane (HMDS)) was used as inorganic compound particles.

[Comparative Example 2]

Predetermined silicone resin sheets were obtained after performing a treatment similarly to Example 1 except that 30 parts by mass of titanium oxide (specific gravity of 4.2, average primary particle diameter of 28 nm, and surface treatment using hexamethyldisilazane (HMDS)) was used as inorganic compound particles.

[Comparative Example 3]

Predetermined silicone resin sheets were obtained after performing a treatment similarly to Example 1 except that 30 parts by mass of iron oxide (specific gravity of 5.2, average primary particle diameter of 42 nm, and surface treatment using hexamethyldisilazane (HMDS)) was used as inorganic compound particles.

[Comparative Example 4]

Predetermined silicone resin sheets were obtained after performing a treatment similarly to Example 1 except that 30 parts by mass of zinc oxide (specific gravity of 5.6, average primary particle diameter of 30 nm, and surface treatment using hexamethyldisilazane (HMDS)) was used as inorganic compound particles.

[Comparative Example 5]

Predetermined silicone resin sheets were obtained after performing a treatment similarly to Example 1 except that 30 parts by mass of zirconium oxide (specific gravity of 5.9, average primary particle diameter of 31 nm, and surface treatment using hexamethyldisilazane (HMDS)) was used as inorganic compound particles.

[Comparative Example 6]

Predetermined silicone resin sheets were obtained after performing a treatment similarly to Example 1 except that 30 parts by mass of barium oxide (specific gravity of 6.7, average primary particle diameter of 52 nm, and surface treatment using hexamethyldisilazane (HMDS)) was used as inorganic compound particles.

[Comparative Example 7]

Predetermined silicone resin sheets were obtained after performing a treatment similarly to Example 1 except that 30 parts by mass of tin oxide (specific gravity of 7.0, average primary particle diameter of 75 nm, and surface treatment using hexamethyldisilazane (HMDS)) was used as inorganic compound particles.

[Comparative Example 8]

Predetermined silicone resin sheets were obtained after performing a treatment similarly to Example 1 except that 30 parts by mass of ytterbium oxide (specific gravity of 9.2, average primary particle diameter of 25 nm, and surface treatment using hexamethyldisilazane (HMDS)) was used as inorganic compound particles.

[Comparative Example 9]

Predetermined silicone resin sheets were obtained similarly to Example 1 such that 98 parts by mass of vinyl terminated polydimethylsiloxane (trade name of "DMS-V42" with a mass average molecular weight of 72,000, manufactured by GELEST, INC.) 2 parts by mass of a methylhydrosiloxane-dimethylsiloxane copolymer (trade name of "HMS-301" with a mass average molecular weight of 2,000 and a proportion of methylhydrosiloxane of 27 mol%, manufactured by GELEST, INC.) were mixed with each other similarly to Example 1 and the mixture was thermally vulcanized using a platinum catalyst.

<Evaluation of Mechanical Strength and Ultrasonic Characteristics>

The following evaluation was performed on silicone resin sheets of Examples 1 to 17 and Comparative Examples 1 to 9.

[Hardness]

The type A durometer hardness of each of the obtained silicone resin sheets with a thickness of 2 mm was measured using a rubber hardness meter (trade name "RH-201A" manufactured by Excel co., Ltd.) in compliance with JIS K6253-3 (2012).

[Tensile Test]

A dumbbell-like test piece of each of the obtained silicone resin sheets with a thickness of 1 mm was manufactured and the tensile strength at break and the tensile elongation at break were measured in compliance with JIS K6251 (2010).

[Tear Strength Test]

A trouser-type test piece of a silicone resin sheet with a thickness of 2 mm was manufactured and the tear strength was measured in compliance with JIS K6252 (2007).

[Abrasion Resistance Test]

A Taber abrasion test was performed on each of the obtained silicone resin sheets with a thickness of 2 mm and mass decrement was measured in compliance with JIS K6264-2 (2005). The measurements were performed under the conditions of a polishing wheel of H22, a load of 9.8 N, and a test rotation speed of 1,000 rotations. The mass decrement less than 30 mg was set to "A", the mass decrement greater than or equal to 30 mg and less than 50 mg was set to "B", the mass decrement greater than or equal to 50 mg and less than 70 mg was set to "C", and the mass decrement greater than 70 mg was set to "D".

Here, "A" and "B" of the evaluation indicate that the sheet has considerably excellent abrasion resistance, "C"

indicates that the sheet is in a usable state, and "D" indicates that the sheet is in an unusable state.

[Acoustic Impedance]

The density of each of the obtained silicone resin sheets with a thickness of 2 mm at 25°C was measured using an electronic gravimeter ("SD-200L" manufactured by ALFA MIRAGE) in accordance with a density measurement method of a method A (underwater substitution method) disclosed in JIS K7112 (1999). The acoustic velocity of an ultrasonic wave was measured at 25°C using a sing-around type acoustic velocity measurement apparatus ("UVM-2 type" manufactured by Ultrasonic Engineering Co., LTd.) in compliance with JIS Z2353 (2003) and acoustic impedance was obtained from a sum of the density and the acoustic velocity which had been measured.

[Acoustic (Ultrasonic) Sensitivity]

A sinusoidal signal (a wave) of 5 MHz which had been output from an ultrasonic oscillator (a function generator with a trade name of "FG-350" manufactured by IWATSU ELECTRIC CO.,LTD.) was input into an ultrasound probe (manufactured by JAPAN PROBE), and an ultrasonic pulse wave with a center frequency of 5 MHz was generated in water from the ultrasound probe. The magnitude of the amplitude before and after the generated ultrasonic wave passed through each of the obtained silicone resin sheets with a thickness of 2 mm was measured in a water temperature environment of 25°C using an ultrasonic receiver (an oscilloscope with a trade name of "VP-5204A" manufactured by Matsushita Electric Industrial Co., LTd.). The acoustic (ultrasonic) attenuation of each sheet was compared with each other by comparing the acoustic (ultrasonic) sensitivities of each sheet.

The acoustic (ultrasonic) sensitivity was regarded as a numerical value given by the following calculation equation.

In the following calculation equation, Vin represents a voltage peak value of a wave with a half-width of less than or equal to 50 nsec which has been input using the ultrasonic oscillator. Vs represents a voltage value obtained in a case where the generated acoustic (ultrasonic) wave passes through the sheet and the ultrasonic oscillator receives the acoustic (ultrasonic) wave which has been reflected from a surface facing the sheet.

$$\text{Acoustic (ultrasonic) sensitivity} = 20 \times \text{Log}\,(\text{Vs} / \text{Vin})$$

The following Tables 1 to 3 are shown after summarizing the obtained results (Example 9 being not according to the invention, as well as the comparative examples).

In the following Tables 1 to 3, the mass average molecular weight of polyorganosiloxane (A) and polyorganosiloxane (B) is described as a molecular weight and the type of each component is described using a trade name.

[Table 1]

| | | | | Example 1 | Example 2 | Example 3 | Example 4 | Example 5 | Example 6 | Example 7 | Example 8 |
|---|---|---|---|---|---|---|---|---|---|---|---|
| Mixture composition | Inorganic compound particles (C) | Type | | Magnesium oxide | Titanium oxide | Iron oxide | Zinc oxide | Zirconium oxide | Barium oxide | Tin oxide | Ytterbium oxide |
| | | Specific gravity | | 3.6 | 4.2 | 5.2 | 5.6 | 5.9 | 6.7 | 7 | 9.2 |
| | | Average primary particle diameter [nm] | | 19 | 14 | 21 | 11 | 11 | 24 | 22 | 20 |
| | | Surface treatment | | HMDS | HMDS | HMDS | HMDS | HMDS | HMDS | HMDS | HMDS |
| | | Content [mass%] | | 30 | 30 | 30 | 30 | 30 | 30 | 30 | 30 |
| | Polyorganosiloxane | Com pone nt (A) | Type | DMS-V42 | DMS-V42 | DMS-V42 | DMS-V42 | DMS-V42 | DMS-V42 | DMS-V42 | DMS-V42 |
| | | | Molecular weight | 72,000 | 72,000 | 72,000 | 72,000 | 72,000 | 72,000 | 72,000 | 72,000 |
| | | | Content [mass%] | 68 | 68 | 68 | 68 | 68 | 68 | 68 | 68 |
| | | Com pone nt (B) | Type | HMS-301 | HMS-301 | HMS-301 | HMS-301 | HMS-301 | HMS-301 | HMS-301 | HMS-301 |
| | | | Molecular weight | 2,000 | 2,000 | 2,000 | 2,000 | 2,000 | 2,000 | 2,000 | 2,000 |
| | | | Content [mass%] | 2 | 2 | 2 | 2 | 2 | 2 | 2 | 2 |
| Evaluation | JIS hardness | | | 36 | 30 | 32 | 37 | 32 | 28 | 26 | 38 |
| | Tensile strength at break [MPa] | | | 2.3 | 1.8 | 2.3 | 2.5 | 2.2 | 1.6 | 2.0 | 2.6 |
| | Tensile elongation at break [%] | | | 630 | 660 | 580 | 700 | 640 | 560 | 600 | 650 |
| | Tear strength [N/cm] | | | 16 | 27 | 17 | 31 | 34 | 16 | 21 | 25 |
| | Taber abrasion test | | | B | A | B | A | A | B | B | B |
| | Acoustic impedance [x $10^6$ $kg/m^2/s$] | | | 1.21 | 1.24 | 1.30 | 1.31 | 1.30 | 1.32 | 1.33 | 1.35 |
| | Acoustic (ultrasonic) sensitivity [dB] | | | -69.5 | -68.3 | -70.6 | -67.5 | -68.1 | -70.4 | -70.0 | -68.0 |

[Table 2]

| | | | | Example 9 | Example 10 | Example 11 Example 12 | | Example 13 | Example 14 | Example 15 | Example 16 | Example 17 . |
|---|---|---|---|---|---|---|---|---|---|---|---|---|
| Mixture composition | Inorganic compound particles (C) | Type | | Zinc oxide | Zinc oxide | Zinc oxide | Zinc oxide | Zinc oxide | Zinc oxide | Zinc oxide | Ytterbium oxide | Ytterbium oxide |
| | | Specific gravity | | 5.6 | 5.6 | 5.6 | 5.6 | 5.6 | 5.6 | 5.6 | 9.2 | 9.2 |
| | | Average primary particle diameter [nm] | | 11 | 11 | 11 | 11 | 11 | 11 | 11 | 20 | 20 |
| | | Surface treatment | | None | HMDS | HMDS | HMDS | HMDS | HMDS | HMDS | HMDS | HMDS |
| | | Content [mass%] | | 30 | 30 | 30 | 30 | 30 | 20 | 40 | 30 | 30 |
| | Polyorganosiloxane | Component (A) | Type | DMS-V42 | DMS-V31 | DMS-V35 | DMS-V46 | DMS-V52 | DMS-V42 | DMS-V42 | PDV-0535 | PDV-1635 |
| | | | Molecular weight | 72,000 | 28,000 | 49,500 | 117,000 | 155,000 | 72,000 | 72,000 | 47,500 | 35,300 |
| | | | Content [mass%] | 68 | 65 | 67 | 69 | 69 | 78 | 59 | 68 | 68 |
| | | Component (B) | Type | HMS-301 | HMS-301 | HMS-301 | HMS-301 | HMS-301 | HMS-301 | HMS-301 | HMS-301 | HMS-301 |
| | | | Molecular weight | 2,000 | 2,000 | 2,000 | 2,000 | 2,000 | 2,000 | 2,000 | 2,000 | 2,000 |
| | | | Content [mass%] | 2 | 5 | 3 | 1 | 1 | 2 | 1 | 2 | 2 |
| Evaluation | JIS hardness | | | 35 | 41 | 39 | 35 | 32 | 30 | 45 | 30 | 35 |
| | Tensile strength at break [MPa] | | | 22 | 2.7 | 2.8 | 2.1 | 2.0 | 2.1 | 2.7 | 2.0 | 2.1 |
| | Tensile elongation at break [%] | | | 650 | 600 | 650 | 670 | 640 | 630 | 650 | 510 | 550 |
| | Tear strength [N/cm] | | | 23 | 17 | 25 | 64 | 71 | 21 | 45 | 16 | 18 |
| | Taber abrasion test | | | A | A | A | A | A | A | A | B | B |
| | Acoustic impedance [x $10^6$ $kg/m^2/s$ | | | 1.30 | 1.31 | 1.31 | 1.31 | 1.31 | 1.20 | 1.35 | 1.52 | 1.55 |
| | Acoustic (ultrasonic) sensitivity [dB] | | | -68.5 | -68.0 | -68.1 | -67.5 | -67.8 | -67.6 | -67.7 | -68.5 | -68.7 |

[Table 3]

| | | | | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 |
|---|---|---|---|---|---|---|---|---|---|---|---|---|
| Mixture composition | Inorganic compound particles (C) | Type | | Magnesium oxide | Titanium oxide | Iron oxide | Zinc oxide | Zirconium oxide | Barium oxide | Tin oxide | Ytterbium oxide | - |
| | | Specific gravity | | 3.6 | 4.2 | 5.2 | 5.6 | 5.9 | 6.7 | 7 | 9.2 | - |
| | | Average primary particle diameter [nm] | | 60 | 28 | 42 | 30 | 31 | 52 | 75 | 25 | - |
| | | Surface treatment | | HMDS | HMDS | HMDS | HMDS | HMDS | HMDS | HMDS | HMDS | - |
| | | Content [mass%] | | 30 | 30 | 30 | 30 | 30 | 30 | 30 | 30 | 0 |
| | Polyorganosiloxane | Component (A) | Type | DMS-V42 | DMS-V42 | DMS-V42 | DMS-V42 | DMS-V42 | DMS-V42 | DMS-V42 | DMS-V42 | DMS-V42 |
| | | | Molecular weight | 72,000 | 72,000 | 72,000 | 72,000 | 72,000 | 72,000 | 72,000 | 72,000 | 72,000 |
| | | | Content [mass%] | 68 | 68 | 68 | 68 | 68 | 68 | 68 | 68 | 98 |
| | | Component (B) | Type | HMS-301 | HMS-301 | HMS-301 | HMS-301 | HMS-301 | HMS-301 | HMS-301 | HMS-301 | HMS-301 |
| | | | Molecular weight | 2,000 | 2,000 | 2,000 | 2,000 | 2,000 | 2,000 | 2,000 | 2,000 | 2,000 |
| | | | Content [mass%] | 2 | 2 | 2 | 2 | 2 | 2 | 2 | 2 | 2 |
| Evaluation | JIS hardness | | | 37 | 32 | 37 | 41 | 34 | 29 | 28 | 41 | 10 |
| | Tensile strength at break [MPa] | | | 2.5 | 2.1 | 2.4 | 2.8 | 2.3 | 2.1 | 2.4 | 2.9 | 0.4 |
| | Tensile elongation at break [%] | | | 450 | 450 | 410 | 460 | 480 | 430 | 400 | 470 | 480 |
| | Tear strength [N/cm] | | | 5 | 11 | 5 | 7 | 9 | 6 | 4 | 12 | 1 |
| | Taber abrasion test | | | D | C | D | D | C | D | D | C | D |
| | Acoustic impedance [x $10^6$ kg/m$^2$/s] | | | 1.22 | 1.23 | 1.26 | 1.28 | 1.28 | 1.31 | 1.33 | 1.34 | 1.00 |
| | Acoustic (ultrasonic) sensitivity [dB] | | | -75.0 | -71.5 | -73.8 | -72.5 | -72.6 | -74.5 | -74.4 | -71.4 | -62.5 |

As shown in Tables 1 to 3, it was possible to obtain high resin hardness, high tensile strength at break, high tensile elongation at break, high tear strength, and excellent abrasion resistance of each silicone resin for an acoustic wave probe of Examples 1 to 17 while maintaining acoustic (ultrasonic) sensitivity of all of the silicone resins within a range greater than or equal to -72 dB. In contrast, in all of the silicone resins for an acoustic wave probe of Comparative Examples 1 to 9, sufficient tensile elongation at break and sufficient tear strength were not obtained.

From the results, it was found that the composition for an acoustic wave probe of the present invention was useful for medical members. In addition, the silicone resin of the present invention can suitably be used in an acoustic wave measurement apparatus, an ultrasound diagnostic apparatus, and an acoustic lens and/or an acoustic matching layer for an acoustic wave probe. Particularly, the composition for an acoustic wave probe and the silicone resin for an acoustic wave probe can suitably be used in an ultrasound probe, in which cMUT is used as an ultrasonic diagnostic transducer array, a photoacoustic wave measurement apparatus, and an ultrasound endoscope for the purpose of improving the sensitivity.

The present invention has been described using an embodiment thereof. However, it is considered that, unless otherwise specified, even the detailed description of the invention is not limited and is necessarily widely interpreted without departing from the attached Claims.

1: acoustic lens
2: acoustic matching layer
3: piezoelectric element layer
4: backing material
7: housing
9: cord
10: ultrasound probe

## Claims

**1.** A use of a composition in the production of an acoustic wave probe, the composition comprising:

a polysiloxane mixture containing polysiloxane having a vinyl group, polysiloxane having two or more Si-H groups in a molecular chain, and one or more inorganic compound particles,
wherein the average primary particle diameter of the inorganic compound particles is less than 25 nm which is measured by the method as described in the description and the inorganic compound particles are selected from the group consisting of magnesium oxide, titanium oxide, iron oxide, zinc oxide, zirconium oxide, barium oxide, tin oxide, and ytterbium oxide, wherein the inorganic compound particles are subjected to surface treatment using a silane compound.

**2.** The use of a composition according to claim 1,
wherein 10 to 60 parts by mass of the inorganic compound particles having an average primary particle diameter of less than 25 nm are contained in 100 parts by mass in total of the polysiloxane mixture.

**3.** The use of a composition according to claim 1 or 2,
wherein 10 to 99.4 parts by mass of the polysiloxane having a vinyl group and 0.5 to 90 parts by mass of the polysiloxane having two or more Si-H groups in a molecular chain are contained in 100 parts by mass in total of the polysiloxane mixture.

**4.** The use of a composition according to any one of claims 1 to 3,
wherein the mass average molecular weight of the polysiloxane having a vinyl group is 20,000 to 200,000, wherein the mass average molecular weight is measured by gel permeation chromatography, which is expressed in terms of polystyrene.

**5.** The use of a composition according to any one of claims 1 to 4,
wherein the mass average molecular weight of the polysiloxane having a vinyl group is 40,000 to 150,000, wherein the mass average molecular weight is measured by gel permeation chromatography, which is expressed in terms of polystyrene.

**6.** The use of a composition according to any one of claims 1 to 5, further comprising:
0.00001 to 0.05 parts by mass of platinum or a platinum compound with respect to 100 parts by mass of the

polysiloxane mixture.

7. A use of a silicone resin in the production of an acoustic wave probe, the resin being obtained by cross-linking the composition according to any one of claims 1 to 6.

8. An acoustic wave probe comprising:

   an acoustic lens formed of the silicone resin for an acoustic wave probe according to claim 7; and/or
   an acoustic matching layer formed of the silicone resin for an acoustic wave probe according to claim 7.

9. An ultrasound probe comprising:

   a capacitive micromachined ultrasonic transducer as an ultrasonic transducer array; and
   an acoustic lens containing the silicone resin for an acoustic wave probe according to claim 7.

10. An acoustic wave measurement apparatus comprising:
    the acoustic wave probe according to claim 8.

11. An ultrasound diagnostic apparatus comprising:
    the acoustic wave probe according to claim 8.

12. A photoacoustic wave measurement apparatus comprising:
    an acoustic lens containing the silicone resin for an acoustic wave probe according to claim 7.

13. An ultrasound endoscope comprising:
    an acoustic lens containing the silicone resin for an acoustic wave probe according to claim 7.

**Patentansprüche**

1. Verwendung einer Zusammensetzung in der Herstellung einer Schallwellensonde, wobei die Zusammensetzung umfasst:

   eine Polysiloxanmischung, umfassend Polysiloxan mit einer Vinylgruppe, Polysiloxan mit zwei oder mehr Si-H-Gruppen in einer molekularen Kette und einen oder mehr anorganische Verbindungspartikel,
   worin der mittlere Primärpartikeldurchmesser der anorganischen Verbindungspartikel kleiner als 25 nm ist, der mit dem wie in der Beschreibung angegebenen Verfahren gemessen wird, und worin die anorganischen Verbindungspartikel ausgewählt sind aus der Gruppe, bestehend aus Magnesiumoxid, Titanoxid, Eisenoxid, Zinkoxid, Zirkoniumoxid, Bariumoxid, Zinnoxid und Ytterbiumoxid, worin die anorganischen Verbindungspartikel einer Oberflächenbehandlung unter Einsatz einer Silanverbindung unterzogen sind.

2. Verwendung einer Zusammensetzung gemäß Anspruch 1,
   worin 10 bis 60 Masseteile der anorganischen Verbindungspartikel mit einem mittleren Primärpartikeldurchmesser von kleiner als 25 nm in insgesamt 100 Masseteilen der Polysiloxanmischung enthalten sind.

3. Verwendung einer Zusammensetzung gemäß Anspruch 1 oder 2,
   worin 10 bis 99,4 Masseteile des Polysiloxans mit einer Vinylgruppe und 0,5 bis 90 Masseteile des Polysiloxans mit zwei oder mehr Si-H-Gruppen in einer molekularen Kette in insgesamt 100 Masseteilen der Polysiloxanmischung enthalten sind.

4. Verwendung einer Zusammensetzung gemäß irgendeinem der Ansprüche 1 bis 3,
   worin das gewichtsgemittelte Molekulargewicht des Polysiloxans mit einer Vinylgruppe 20.000 bis 200.000 beträgt, worin das gewichtsgemittelte Molekulargewicht durch Gelpermeationschromatographie gemessen wird, welches ausgedrückt ist als Polystyrol.

5. Verwendung einer Zusammensetzung gemäß irgendeinem der Ansprüche 1 bis 4,
   worin das gewichtsgemittelte Molekulargewicht des Polysiloxans mit einer Vinylgruppe 40.000 bis 150.000 beträgt, worin das gewichtsgemittelte Molekulargewicht gemessen wird durch Gelpermeationschromatographie, welches

ausgedrückt ist als Polystyrol.

6. Verwendung einer Zusammensetzung gemäß irgendeinem der Ansprüche 1 bis 5, ferner umfassend: 0,00001 bis 0,05 Masseteile von Platin oder einer Platinverbindung, bezogen auf 100 Masseteile der Polysiloxanmischung.

7. Verwendung eines Silikonharzes in der Herstellung einer Schallwellensonde, wobei das Harz erhalten ist durch Vernetzen der Zusammensetzung gemäß irgendeinem der Ansprüche 1 bis 6.

8. Schallwellensonde, umfassend:

eine akustische Linse, gebildet aus dem Silikonharz für eine Schallwellensonde gemäß Anspruch 7; und/oder
eine akustische Angleichschicht, gebildet aus dem Silikonharz für eine Schallwellensonde gemäß Anspruch 7.

9. Ultraschallsonde, umfassend:

einen kapazitiven, mikrobearbeiteten Ultraschallwandler als Ultraschallwandleranordnung; und
eine akustische Linse, enthaltend das Silikonharz für eine Schallwellensonde gemäß Anspruch 7.

10. Schallwellen-Messvorrichtung, umfassend:
die Schallwellensonde gemäß Anspruch 8.

11. Ultraschall-Diagnosevorrichtung, umfassend:
die Schallwellensonde gemäß Anspruch 8.

12. Messvorrichtung für fotoakustische Wellen, umfassend:
eine akustische Linse, enthaltend das Silikonharz für eine Schallwellensonde gemäß Anspruch 7.

13. Ultraschall-Endoskop, umfassend:
eine akustische Linse, enthaltend das Silikonharz für eine Schallwellensonde gemäß Anspruch 7.

**Revendications**

1. Utilisation d'une composition dans la production d'une sonde à ondes acoustiques, la composition comprenant :

un mélange de polysiloxanes contenant un polysiloxane présentant un groupe vinyle, un polysiloxane présentant deux groupes Si-H ou plus dans une chaîne moléculaire, et une ou plusieurs particules de composés inorganiques,
dans laquelle le diamètre primaire moyen des particules de composés inorganiques est inférieur à 25 nm, mesuré par le procédé décrit dans la description, et les particules de composés inorganiques sont sélectionnées dans le groupe constitué par l'oxyde de magnésium, l'oxyde de titane, l'oxyde de fer, l'oxyde de zinc, l'oxyde de zirconium, l'oxyde de baryum, l'oxyde d'étain, et l'oxyde d'ytterbium, dans laquelle les particules de composés inorganiques sont soumises à un traitement de surface en utilisant un composé de silane.

2. Utilisation d'une composition selon la revendication 1,
dans laquelle 10 à 60 parties en masse des particules de composés inorganiques présentant un diamètre primaire moyen des particules inférieur à 25 nm sont contenues dans 100 parties en masse au total du mélange de polysiloxanes.

3. Utilisation d'une composition selon la revendication 1 ou la revendication 2,
dans laquelle 10 à 99,4 parties en masse du polysiloxane présentant un groupe vinyle et 0,5 à 90 parties en masse du polysiloxane présentant deux groupes Si-H ou plus dans une chaîne moléculaire sont contenues dans 100 parties en masse au total du mélange de polysiloxanes.

4. Utilisation d'une composition selon l'une quelconque des revendications 1 à 3,
dans laquelle le poids moléculaire moyen en masse du polysiloxane présentant un groupe vinyle est entre 20 000 et 200 000, dans laquelle le poids moléculaire moyen en masse est mesuré par chromatographie par perméation de gel

et exprimé en termes de polystyrène.

**5.** Utilisation d'une composition selon l'une quelconque des revendications 1 à 4, dans laquelle le poids moléculaire moyen en masse du polysiloxane présentant un groupe vinyle est entre 40 000 et 150 000, dans laquelle le poids moléculaire moyen en masse est mesuré par chromatographie par perméation de gel et exprimé en termes de polystyrène.

**6.** Utilisation d'une composition selon l'une quelconque des revendications 1 à 5, comprenant en outre : 0,00001 à 0,05 partie en masse de platine ou d'un composé de platine par rapport à 100 parties en masse du mélange de polysiloxanes.

**7.** Utilisation d'une résine de silicone dans la fabrication d'une sonde à ondes acoustiques, la résine étant obtenue par réticulation de la composition selon l'une quelconque des revendications 1 à 6.

**8.** Sonde à ondes acoustiques comprenant :

une lentille acoustique formée de la résine de silicone pour une sonde à ondes acoustiques selon la revendication 7 ; et/ou
une couche d'adaptation acoustique formée de la résine de silicone pour une sonde à ondes acoustiques selon la revendication 7.

**9.** Sonde à ultrasons comprenant :

un transducteur ultrasonique capacitif micro-usiné en tant que réseau de transducteurs ultrasoniques ; et
une lentille acoustique contenant la résine de silicone pour une sonde à ondes acoustiques selon la revendication 7.

**10.** Appareil de mesure d'ondes acoustiques comprenant : la sonde à ondes acoustiques selon la revendication 8.

**11.** Appareil de diagnostic par ultrasons comprenant : la sonde à ondes acoustiques selon la revendication 8.

**12.** Appareil de mesure d'ondes photoacoustiques comprenant : une lentille acoustique contenant la résine de silicone pour une sonde à ondes acoustiques selon la revendication 7.

**13.** Endoscope à ultrasons comprenant : une lentille acoustique contenant la résine de silicone pour une sonde à ondes acoustiques selon la revendication 7.

FIG. 1

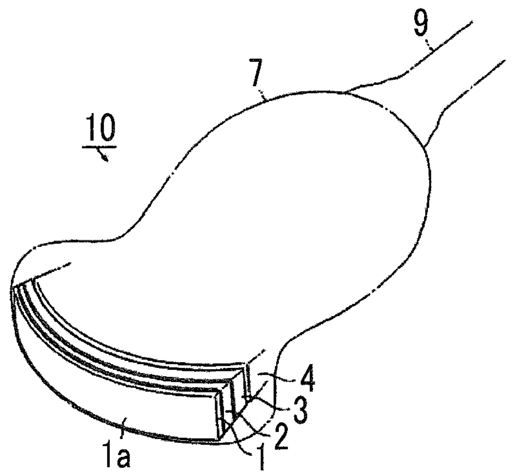

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 62011897 A
- JP S62011897 A
- US 2009243436 A1
- JP 2005125071 A
- JP 2005253751 A
- JP 2003169802 A
- JP 2013202050 A
- JP 2013188465 A
- JP 2013180330 A
- JP 2013158435 A
- JP 2013154139 A
- JP 2011071842 A
- JP 2006157320 A
- JP 2008311700 A